# EUROPÄISCHE PATENTSCHRIFT

(11) **EP 3 007 628 B1**
(45) Veröffentlichungstag und Bekanntmachung des Hinweises auf die Patenterteilung: **16.08.2017**
(21) Anmeldenummer: 15710195.7
(22) Anmeldetag: 17.03.2015
(51) Int. Cl.: A61B 17/00

(54) **STEUERUNG ZUR SICHEREN MONTAGE UND DEMONTAGE ZWEIER FUNKTIONSEINHEITEN EINES MEHRTEILIGEN MEDIZINTECHNISCHEN GERÄTS**
CONTROL FOR RELIABLE ASSEMBLY AND DISASSEMBLY OF TWO FUNCTIONAL UNITS OF A MULTI-PART MEDICAL DEVICE
COMMANDE PERMETTANT DE MONTER ET DÉMONTER DE MANIÈRE SÛRE DEUX UNITÉS FONCTIONNELLES D'UN ÉQUIPEMENT MÉDICAL EN PLUSIEURS PARTIES

(30) Priorität: 26.03.2014 DE 102014104179
(43) Veröffentlichungstag der Anmeldung: 20.04.2016
(73) Patentinhaber: Aesculap AG, 78532 Tuttlingen (DE)
(72) Erfinder: VOGTHERR, Robert, 78532 Tuttlingen (DE); MASER, Thomas, 78658 Zimmern ob Rottweil (DE); REICHLE, Heiko, 78532 Tuttlingen (DE); SEYFRIED, Dominik, 78126 Königsfeld (DE); KELLER, Anton, 78589 Dürbheim (DE)
(74) Vertreter: Winter, Brandl, Fürniss, Hübner, Röss, Kaiser, Polte - Partnerschaft mbB
(86) Internationale Anmeldenummer: PCT/EP2015/055582
(87) Internationale Veröffentlichungsnummer: WO 2015/144501

(56) Entgegenhaltungen:
- WO-A1-97/16123
- WO-A1-2013/181100
- WO-A2-2007/027470
- DE-A1-102006 033 439
- DE-B3-102006 001 677
- JP-A- 2009 000 426

## Beschreibung

Die vorliegende Erfindung betrifft ein mehrteiliges medizintechnisches Gerät mit zwei miteinander mechanisch koppelbaren Geräteeinheiten und zwei miteinander mechanisch koppelbaren Funktionseinheiten gemäß dem Oberbegriff des Anspruchs 1 sowie ein entsprechendes Verfahren gemäß dem Oberbegriff des nebengeordneten Anspruchs. Genauer gesagt betrifft die vorliegende Erfindung ein gattungsgemäßes medizintechnisches Gerät mit einer ersten Funktionseinheit und einer zweiten Funktionseinheit, wobei im mechanisch gekoppelten Zustand mit der ersten Funktionseinheit die zweite Funktionseinheit kraftbetätigt werden kann, die erste Funktionseinheit elektrisch oder elektronisch ansteuerbar ist und einen mechanischen Kopplungsabschnitt aufweist, der zum Koppeln mit einem entsprechenden mechanischen Kopplungsabschnitt der zweiten Funktionseinheit in eine Kopplungsposition bringbar ist.

Bei vielen chirurgischen Eingriffen werden heutzutage kraftbetätigte Geräte verwendet, die bestimmte Arbeiten oder Bewegungen ausführen oder steuern können. Als Beispiel sei eine chirurgische Vorrichtung zum Stabilisieren oder Immobilisieren eines Teils eines bewegten Gewebes oder auch zum Positionieren von Organen oder chirurgischen Instrumenten und Geräten während einer Operation mittels eines beweglichen Haltearms erwähnt. Ein solcher beweglicher Haltearm kann mittels Druckluft aus seinem arretierten Zustand in einen beweglichen Zustand gebracht werden kann. Die Steuerung der Druckluft erfolgt dabei mittels einer Steuerelektronik, welcher die Signale von Betätigungselementen und Sensoren zugeführt wird.

Weiterhin bestehen diverse Instrumente und Geräte aus einem zweiteiligen Aufbau, da einerseits ein Teil des Gerätes aufgrund der Geometrie, der verwendeten Materialien oder ähnlichem nicht aufbereitet und sterilisiert werden kann, oder einem derart hohen Verschleiß unterliegt, dass es nur für den Einmal-Einsatz vorgesehen wird, und andererseits ein weiterer Teil des Gerätes derart kostenintensive Bauteile aufweist, dass ein Einmal-Einsatz wirtschaftlich nicht sinnvoll ist.

Die Kombination dieser Zweiteilung mit einem kraftbetätigten oder anderweitig fremdenergetisch gesteuerten oder arbeitenden Gerät ergibt diverse technische Probleme, die aus der Montage und Demontage oder der Verbindung der Steuer- oder Arbeitseinrichtungen von beiden Teilen des Gerätes resultieren.

Bei dem erwähnten Beispiel müssen zwei Teile, nämlich eine Technikeinheit, die die kraftbetätigte Arretierung des beweglichen Haltearmes ermöglicht, und der Haltearm selbst miteinander verbunden werden. Beide Teile des Gerätes verfügen über jeweils ein Betätigungselement, mit dem ein Schieber in der Technikeinheit verfahren werden kann. Die Betätigungselemente haben dieselbe Funktion. Bei Betätigung eines dieser Betätigungselemente im adaptierten Zustand des Haltearms verfährt der Schieber gegen eine Druckfeder nach vorn aus dem Gehäuse heraus. Werden die Betätigungselemente dagegen nicht betätigt, wird der Schieber durch eine Federkraft nach hinten ins Gehäuse der Technikeinheit hineingedrückt. Beide Betätigungselemente sind als Taster ausgeführt. Dadurch muss der Chirurg während der Anwendung nicht zwischen Aktivieren und Deaktivieren des Arbeitsvorganges hin- und her schalten. Die Rückstellung des Schiebers und damit die Arretierung des Haltearmes geschieht, sobald das Betätigungselement losgelassen wird, durch die Federkraft der Druckfeder automatisch.

Die Betätigungselemente werden ebenfalls zum Montieren und Demontieren der beiden Teile benötigt: Mit dem auf der Technikeinheit befindlichen Betätigungselement wird der Schieber nach vorn verfahren, dann kann der Haltearm adaptiert werden. Wird das Betätigungselement losgelassen, wird der Halterarm nun erstmals arretiert. Danach kann mit dem gesamten Gerät und beiden Betätigungselementen gearbeitet werden. Zur Demontage wird wiederum das Betätigungselement der Technikeinheit aktiviert, der Schieber verfahren und der Haltearm kann von der Technikeinheit abgenommen werden.

Zum einen sind diese Vorgänge nicht komfortabel. Da das Betätigungselement als Taster ausgeführt ist, darf es während der Montage- oder Demontagephase nicht losgelassen werden. Wird das elektrische Signal des Betätigungselementes unterbrochen, beispielsweise weil sich der Anwender während der Montage- oder Demontagephase kurz unbewusst bewegt, fährt der Schieber sofort ins Gehäuse der Technikeinheit hinein. Ist der Anschluss des Haltearms zu diesem Zeitpunkt noch nicht korrekt im Schieber adaptiert, können Bauteile von Technikeinheit oder Haltearm durch das Zurückfahren beschädigt werden. Würde der Anwender während der Montage- oder Demontagephase kurzzeitig beide Hände benötigen, weil etwas klemmt und sich der Adaptionsvorgang schwergängig gestaltet, ist ihm das nicht möglich, da er das Betätigungselement nicht loslassen darf.

Zum Anderen sind die Montage- und Demontagephase mit einem erheblichen Risiko für den Anwender behaftet. Solange er mit einer Hand das Betätigungselement auf der Technikeinheit in aktivierter Stellung hält, ist der Schieber ausgefahren und nicht mehr vom Gehäuse verdeckt. Der Schieber besitzt eine nach oben geöffnete Geometrie, damit das Adaptieren des Anschlusses des Haltearmes ermöglicht wird. Ein seitliches Einschieben oder andere Lösungen um das Risiko dieser Geometrie zu verringern, scheinen hierbei nicht praktikabel. In diese offene Geometrie des Schiebers könnte der Anwender beispielsweise mit seinem Finger hineingreifen, wenn er zum Beispiel Schmutz in dieser Adaptionsstelle entdeckt und entfernen möchte. Würde er nun das Betätigungselement loslassen, fährt der Schieber schlagartig mittels der Federkraft ins Gehäuse der Technikeinheit hinein. Dies kann für den Anwender ein hohes Verletzungsrisiko darstellen.

Ein technisch vergleichbares System stellt die pneumatische Knochenstanze gemäß DE 20 2004 015 643 U1 dar. Dieses Gerät arbeitet pneumatisch und besteht aus dem Handgriff mit allen technischen Einrichtungen, die ein kraftbetätigtes Arbeiten ermöglichen, und einem Stanzenschaft, der in den Handgriff eingelegt, beziehungsweise mit ihm adaptiert werden muss. Bei diesem System werden ein Sicherheitshebel und ein Ein-/Ausschalter beworben, die beide das Unfallrisiko, das durch solch ein kraftbetätigtes System resultiert, verringern. Der Sicherheitshebel blockiert den Mechanismus der Stanze beim Säubern des Stanzenschafts, wenn der Anwender also gewollt in den Arbeitsbereich der Stanze greifen muss. Diese Blockade wird rein mechanisch erzeugt.

Der Ein-/Ausschalter ist im technischen Sinne eigentlich ein Tast-Schalter, der im gedrückten Zustand das Gerät aktiviert und beim Loslassen des Tasters automatisch in den Zustand "Aus" beziehungsweise "Gerät inaktiv" übergeht. Der Ein-/Ausschalter verfügt zusätzlich über einen verdeckt eingebauten Dreh-Schalter. Mittels dieses Zusatz-Schalters kann der Hauptschalter oder -taster blockiert werden, um ein unabsichtliches Betätigen des Geräts, beispielsweise beim Ablegen auf unebenem Untergrund, wodurch der Einschalter durch das Eigengewicht des Geräts aktiviert werden könnte, zu verhindern. Auch diese Blockade wird mechanisch erzeugt.

Die Lösungen, die bei der Knochenstanze gemäß DE 20 2004 015 643 U1 ein gefahrenfreies Arbeiten ermöglichen sollen, benötigen ausreichend Bauraum. Der Sicherheitshebel, der die volle Stanzenkraft abfangen und halten muss, und mit ausreichender Sicherheit konstruiert ist, kann in bestimmten Geräten, die ebenfalls mit viel Kraft arbeiten aber wesentlich kompakter bauen, nicht immer umgesetzt werden. Der größere Nachteil ist aber, dass dieser Mechanismus nicht automatisch greift und vergessen werden kann.

Aus der WO 2007/027470 A2 ist eine Führungsklammer/ -aufsatz mit einer medizinischen Führungseinrichtung bekannt, die auf ein medizintechnisches Gerät (z.B. Bohrer, Nadel, Säge) aufgesetzt werden kann und mithilfe der Führungseinrichtung dem Anwender hilft, das medizintechnische Gerät, z.B: in einer Operationsstelle, richtig zu platzieren/ zu führen. Führungsklammer und medizintechnisches Gerät weisen eine Positionserkennung auf, die dazu dient zu überprüfen, ob die Führungsklammer richtig auf dem Gerät aufsitzt und korrekt ausgerichtet ist.

Die Druckschrift JP 2009-00426 A offenbart ein Ultraschallgerät, welches einen Stecker und eine Steckdose mit einer automatisch schließ- und offenbaren Blende aufweist.

Der vorliegenden Erfindung liegt somit die Aufgabe zugrunde, ein mehrteiliges medizintechnisches Geräte und ein entsprechendes Verfahren bereitzustellen, das auf eine einfache Weise eine für den Anwender komfortable und sichere Montage und Demontage von zwei miteinander zu koppelnde Funktionseinheiten zweier Geräteinheiten ermöglicht.

Diese Aufgabe wird hinsichtlich des medizintechnischen Geräts durch die Merkmale des Anspruchs 1 und hinsichtlich des Verfahrens durch die Merkmale des nebengeordneten Anspruchs gelöst.

Vorteilhafte Weiterbildungen sind Gegenstand der Unteransprüche.

Erfindungsgemäß wird ein elektronisches Schaltungskonzept bzw. Steuerungsverfahren an einem zweiteiligen medizintechnischen, insbesondere chirurgischen Gerät, das kraftbetätigt arbeitet und elektronisch gesteuert wird, vorgeschlagen, wobei das Steuerungskonzept die Risiken des Geräts, die insbesondere vor oder beim Adaptieren der zweiten Geräteeinheit vorliegen, minimiert. Des Weiteren ermöglicht das Steuerungskonzept das komfortable Zusammenbauen bzw. Adaptieren der beiden Geräte aneinander.

Ein erfindungsgemäßes mehrteiliges medizintechnisches Gerät weist zwei miteinander mechanisch koppelbare Geräteeinheiten auf, wobei die beiden Geräteeinheiten zwei miteinander mechanisch koppelbare Funktionseinheiten aufweisen, um im mechanisch gekoppelten Zustand mit der ersten Funktionseinheit die zweite Funktionseinheit (kraft-)betätigen zu können. Dabei ist die erste Funktionseinheit elektrisch oder elektronisch ansteuerbar und weist einen mechanischen Kopplungsabschnitt auf, der zum Koppeln mit einem entsprechenden mechanischen Kopplungsabschnitt der zweiten Funktionseinheit in eine Kopplungsposition bringbar ist. Erfindungsgemäß ist zumindest an einer Geräteeinheit ein Mittel zum Erfassen einer Relativlage oder eines Abstands der beiden Geräteeinheiten zueinander vorgesehen und die elektrische oder elektronische Ansteuerung der ersten Funktionseinheit ist derart konfiguriert, dass diese nur dann in die Kopplungsposition bringbar ist, wenn sich die beiden Geräteinheiten in einer bestimmten Relativlage zueinander befinden oder einen vorbestimmten Mindestabstand zueinander unterschreiten.

Diese Konfiguration bzw. Schaltlogik des Gerätes minimiert die Risiken für den Anwender, die beim Montieren beziehungsweise Demontieren der beiden Geräteteile entstehen. Die Ansteuerung erfolgt abstandsabhängig und arbeitet somit selbstständig und kann im Gegensatz zu vielen erdenklichen mechanischen Lösungen nicht vergessen oder übersehen werden.

Aufgrund der Einfachheit der Montage und Demontage ist diese auch anwendungsfreundlicher. Für die abstandsabhängige elektrische oder elektronische Ansteuerung der ersten Funktionseinheit können elektrische Bauteile verwendet werden, die im Vergleich zu mechanischen Sicherheitseinrichtungen nur einen geringen Platzbedarf benötigen. Dieser Platz steht bei medizintechnischen Geräten nämlich oftmals nicht zur Verfügung. Andererseits kann das Gerät mit solch einer Schaltung wesentlich kompakter realisiert werden als mit einer vergleichbaren mechanischen Lösung.

Dabei kann die Relativlage oder der Mindestabstand so gewählt sein, dass ein Benutzer mit seiner Hand oder seinem Finger nicht zwischen beide Geräteinheiten greifen kann oder die beiden Geräteeinheiten die beiden Kopplungsabschnitte der Funktionseinheiten derart verdecken, dass ein Benutzer mit seiner Hand oder seinem Finger diese nicht erreichen kann.

Dadurch wird selbst bei einer unvorsichtigen Handhabung des medizintechnischen Geräts sichergestellt, dass der Anwender nicht in den ausgefahrenen bzw. in die Kopplungsposition gebrachten Kopplungsabschnitt greifen kann und beim Einfahren der Kopplungsabschnitts nicht die Finger gequetscht oder anderweitig verletzt werden.

Gemäß einer Konfiguration kann der Mindestabstand kleiner oder gleich 40 mm, insbesondere kleiner oder gleich 20 mm, besonders bevorzugt kleiner oder gleich 10 mm betragen.

Die Mittel zum Erfassen einer Relativlage oder eines Abstands der beiden Geräteeinheiten können als ein am oder zumindest in der Nähe des Kopplungsabschnitts der ersten Funktionseinheit vorgesehener, insbesondere magnetischer, Nährungsschalter oder Abstandssensor realisiert sein, der durch am oder zumindest in der Nähe des Kopplungsabschnitts der zweiten Funktionseinheit vorgesehene Mittel, insbesondere Magneten, auslösbar ist, wenn der erste Kopplungsabschnitt in die Nähe des zweiten Kopplungsabschnitts gebracht wird und der Mindestabstand unterschritten wird. Alternativ von ein an einer Geräteinheit vorgesehenen autarker Abstandsensor verwendet werden, der ohne weitere Mittel an der anderen Geräteeinheit, den Abstand zwischen beiden Geräteeinheiten messen kann, verwendet werden.

Der Nährungssensor bzw. -schalter wird erstmalig bei Einnahme der vorbestimmten Relativlage oder Unterschreiten des vorbestimmten Mindestabstandes der Geräteeinheiten und/oder Funktionseinheiten ausgelöst, aktiviert oder geschaltet, und verbleibt solange in diesem Zustand, bis die Geräteeinheiten bzw. Funktionseinheiten die vorbestimmte Relativlage wieder verlassen oder den Mindestabstand einnehmen oder überschreiten, z.B. bei der Demontage der beiden Geräteeinheiten.

Dies ermöglicht eine berührungslose und automatische Aktivierung des Nährungssensors bzw. -schalters und eine damit verbundene Ansteuerung der ersten Funktionseinheit ohne Zutun des Anwenders.

Der Nährungsschalter oder Abstandssensor kann in der Geräteinheit der ersten Funktionseinheit versenkt oder verdeckt eingebaut sein. Je nach Stärke des Magneten und Sensibilität des Näherungsschalters kann sich auch eine gewisse Materialdicke des Gehäuses der Technikeinheit zwischen den beiden Bauteilen befinden.

Dadurch ergibt sich eine geringere Gefahr des Verschmutzens oder Beschädigens des Näherungssensors oder -schalters. Ferner ist dieser nicht unmittelbar den Einflüssen beim Aufbereiten durch Sterilisation ausgesetzt.

Gemäß einem zusätzlichen oder alternativen Aspekt können beim Koppeln der Geräteeinheiten gleichzeitig die beiden Funktionseinheiten gekoppelt werden.

Dies hat den Vorteil, dass in einer Bewegung bzw. in einem Vorgang sowohl die beiden Geräteeinheiten als auch die beiden zugehörigen Funktionseinheiten miteinander gekoppelt werden. Beim Koppeln der Geräteeinheiten werden dabei die Funktionseinheiten automatisch in eine vorbestimmte Kopplungsposition gebracht.

Die Geräteeinheiten können, wenn sie miteinander mechanisch gekoppelt sind, auch elektrisch und/oder signaltechnisch gekoppelt sein.

Somit werden beim Koppeln der Geräteeinheiten diese nicht nur mechanisch, sondern auch elektrisch und/oder signaltechnisch verbunden, um die zweite Geräteinheit bzw. andere dort vorgesehene Funktionseinheiten ansteuern zu können, aber auch über die zweite Geräteeinheit, z.B. durch entsprechende dort vorgesehene Steuermittel oder Betätigungselemente, auf die erste Geräte- und Funktionseinheit einzuwirken oder diese ansteuern zu können.

Die mechanischen und elektrischen und/oder signaltechnischen Schnittstellen zwischen den beiden Geräteeinheiten können so konfiguriert sein, dass beim Koppeln der beiden Geräteeinheiten die elektrische und/oder signaltechnische Kopplung erst bei vollständiger mechanischer Kopplung erfolgt und beim Entkoppeln der beiden Geräteinheiten die elektrische und/oder signaltechnische Kopplung vor der mechanischen Kopplung gelöst wird.

Dadurch wird erreicht, dass die vollständige mechanische Kopplung bzw. das Lösen der selbigen erkannt wird und eine entsprechende Ansteuerung der Funktionseinheiten bzw. des mechanische Kopplungsabschnitt der ersten Funktionseinheit erfolgen kann. So kann mit der elektrischen und/oder signaltechnischen Kopplung eine automatische Zurückführung des Kopplungsabschnitts in eine vorbestimmte Position, z.B. Ruheposition, einhergehen und/oder beim Lösen der elektrischen und/oder signaltechnischen Kopplung des Kopplungsabschnitts automatisch in die Kopplungs- bzw. Entkopplungsposition gebracht oder ausgefahren werden. Beim Entkoppeln kann der Kopplungsabschnitt ferner solange in der Kopplungsposition gehalten werden, bis der Mindestabstand zwischen beiden Geräteeinheiten überschritten wird, und nach Überschreiten des Mindestabstands automatisch in die vorbestimmte Position gebracht werden.

Der Anwender wird somit nicht mit einer bestimmten Abfolge an vorzunehmenden Betätigungsvorgängen zur Montage und Demontage der Geräte- und Funktionseinheiten behelligt.

Der Kopplungsabschnitt der ersten Funktionseinheit kann in eine vorbestimmte Position federvorgespannt sein und mittels Fremdenergie, insbesondere hydraulischem oder pneumatischen Druck oder mittels eines Elektromotors, in die Kopplungsposition bringbar sein.

Dadurch lässt sich die Ansteuerung der Funktionseinheiten vereinfachen, indem lediglich die Verfahr- oder Betätigungsrichtung in eine Richtung elektrisch aktiv gesteuert werden muss und die Verfahr- oder Betätigungsrichtung in die andere Richtung durch eine mechanische Rückstellung erfolgt. Damit lässt sich die elektrische Betätigung insgesamt vereinfachen. Alternativ können auch die Funktionseinheiten in beide Verfahr- bzw. Betätigungsrichtungen aktiv (hydraulisch, pneumatisch oder elektromotorisch) gesteuert werden.

Im gekoppelten Zustand der beiden Geräteeinheiten kann die erste Funktionseinheit und mittelbar die zweite Funktionseinheit durch Betätigen eines ersten Betätigungselements, das an der ersten Funktionseinheit vorgesehen ist, elektrisch oder elektronisch ansteuerbar sein und im entkoppelten Zustand der beiden Geräteeinheiten kann der Kopplungsabschnitt der ersten Funktionseinheit mittels der Fremdenergie automatisch in die Kopplungsposition gebracht werden, sofern sich die beiden Geräteinheiten in der bestimmten Relativlage zueinander befinden oder den vorbestimmten Mindestabstand zueinander unterschritten haben.

Über lediglich ein Betätigungselement kann somit sowohl der normale Betrieb der Funktionseinheiten als auch der Kopplungs- bzw. Entkopplungsvorgang gesteuert werden. Dadurch lassen sich das Vorsehen zusätzlicher Betätigungselemente und entsprechender Verbindungsleitungen lediglich für das Koppeln und Entkoppeln der Geräte- und Funktionseinheiten und ggf. damit verbundene Fehlbedienungen durch den Anwender vermeiden.

Gemäß einem zusätzlichen oder alternativen Aspekt kann im gekoppelten Zustand der beiden Geräteeinheiten die eine Funktionseinheit und mittelbar die andere Funktionseinheit durch Betätigen eines zweiten Betätigungselements, das an der zweiten Funktionseinheit vorgesehen ist, elektrisch oder elektronisch ansteuerbar sein, und das zweite Betätigungselement derart ausgebildet sein, insbesondere als elektrischer Öffner-Kontakt ausgebildet sein, so dass beim elektrischen Koppeln der beiden Geräteeinheiten die Energiezufuhr für die Ansteuerung der ersten Funktionseinheit, beispielsweise die Energiezufuhr für die Ansteuerung eines Ventils, unterbrochen wird.

Diese Konfiguration hat zwei Vorteile. Zum einen kann die erste Funktionseinheit und mittelbar darüber die zweite Funktionseinheit auch über ein Betätigungselement an der zweiten Geräteinheit angesteuert werden. Gleichzeitig kann die schaltungstechnische Anbindung des zweiten Betätigungselements in dem Gesamtschaltkreis so erfolgen, dass durch die Adaption bzw. elektrische Kopplung der zweiten Geräteeinheit eine vorbestimmte elektrische Steuerung in der ersten Geräteeinheit erfolgt.

Gemäß einem zusätzlichen oder alternativen Aspekt kann im gekoppelten Zustand durch Betätigen des ersten oder des zweiten Betätigungselements der Kopplungsabschnitt in eine vorbestimmte Arbeitsposition gebracht wird. Diese vorbestimmte Arbeitsposition kann der Kopplungs- bzw. Entkopplungsposition entsprechen.

Dadurch kann die Anzahl möglicher Arbeitsstellungen und somit die Ansteuerung der Funktionseinheiten, genauer gesagt der ersten Funktionseinheit, minimiert werden, wodurch das Schaltungskonzept noch einfacher gestaltet werden kann.

Gemäß einem Aspekt der Erfindung kann die erste Geräteeinheit eine Technikeinheit sein, welche mit einer elektrischen und einer Fremdenergiequelle verbunden ist, und die zweite Geräteeinheit ein an der Technikeinheit befestigbarer flexibler Gliederarm sein, der in unterschiedliche Positionen und/oder Lagen bringbar ist. Die erste Funktionseinheit kann ein Spannmechanismus sein und die zweite Funktionseinheit kann ein mit dem Spannmechanismus koppelbares Zugseil sein, das durch Gliederelemente des Gliederarms geführt ist und über welches die Gliederelemente reibschlüssig gegeneinander verspannt werden können, um den Gliederarm in einer gewünschten Stellung feststellbar zu machen.

Dadurch kann das erfindungsgemäße Adaptions- und Schaltungskonzept auf eine chirurgische Vorrichtung zum Stabilisieren oder Immobilisieren von sich bewegenden Organen angewandt werden.

Ein weiterer Aspekt der vorliegenden Erfindung betrifft ein Verfahren zum mechanischen Koppeln von zwei Funktionseinheiten eines zwei- oder mehrteiligen medizintechnisches Geräts, wobei das medizintechnisches Gerät zwei miteinander mechanisch koppelbaren Geräteeinheiten aufweist, die beiden Geräteeinheiten zwei miteinander mechanisch koppelbare Funktionseinheiten aufweisen, um im mechanisch gekoppelten Zustand mit der ersten Funktionseinheit die zweite Funktionseinheit (kraft-)betätigen zu können, und die erste Funktionseinheit elektrisch oder elektronisch ansteuerbar ist und einen mechanischen Kopplungsabschnitt aufweist, der zum Koppeln mit einem entsprechenden mechanischen Kopplungsabschnitt der zweiten Funktionseinheit in eine Kopplungsposition bringbar ist. Erfindungsgemäß weist das Verfahren die folgende Schritte auf: Erfassen einer Relativlage oder eines Abstands der beiden Geräteeinheiten zueinander, und Konfigurieren der elektrischen oder elektronisches Ansteuerung der ersten Funktionseinheit derart, dass diese nur dann in die Kopplungsposition gebracht wird, wenn sich die beiden Geräteinheiten in einer bestimmten Relativlage zueinander befinden oder einen vorbestimmten Mindestabstand zueinander unterschreiten.

Für das erfindungsgemäße Verfahren ergeben sich dieselben im Zusammenhang mit dem erfindungsgemäßen medizintechnischen Gerät beschriebenen Vorteile.

### Kurze Beschreibung der Zeichnungen

Fig. 1 zeigt eine perspektivische Ansicht einer chirurgischen Vorrichtung gemäß einer bevorzugten Ausführungsform der Erfindung;
Fig. 2 zeigt einen Technikblock mit gekoppeltem Gliederarm der in der Fig. 1 gezeigten chirurgischen Vorrichtung;
Fig. 3 zeigt einen Technikblock mit entkoppeltem Gliederarm der in der Fig. 1 gezeigten chirurgischen Vorrichtung;
Fig. 4 zeigt eine Querschnittsansicht des in der Fig. 2 gezeigten Technikblocks;
Fig. 5 zeigt eine perspektivische Ansicht des Gliederarms;
Fig. 6A zeigt eine perspektivische Ansicht des Technikblocks mit einem Schlitten eines Spannmechanismus in seiner Ruheposition;
Fig. 6B zeigt eine perspektivische Ansicht des Technikblocks mit dem Schlitten des Spannmechanismus in seiner Kopplungsposition;
Fig. 7 zeigt ein elektrisches Schaltbild gemäß der bevorzugten Ausführungsform der Erfindung;
Fig. 8 zeigt eine Schaltlogik gemäß der bevorzugten Ausführungsform der Erfindung; und
Fig. 9 zeigt ein Logikdiagramm gemäß der bevorzugten Ausführungsform der Erfindung.

### Detaillierte Beschreibung bevorzugter Ausführungsformen

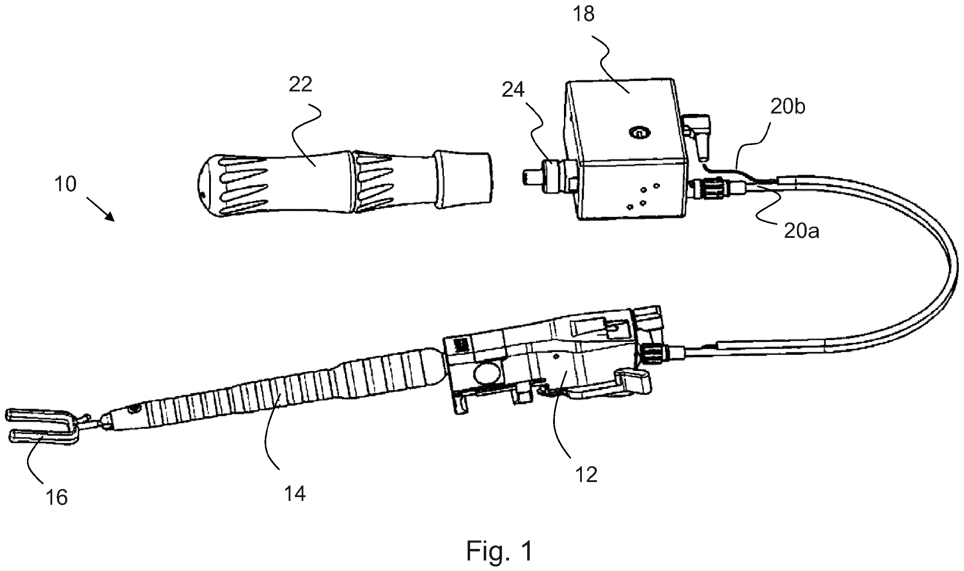

Fig. 1 zeigt eine perspektivische Ansicht eines mehrteiligen medizintechnischen Geräts in Form einer chirurgischen Vorrichtung 10 zum Stabilisieren oder Immobilisieren eines Teils eines bewegten Gewebes oder auch zum Positionieren von Organen gemäß einer ersten Ausführungsform der Erfindung. Die Vorrichtung 10 weist einen Grundkörper oder Technikblock 12 auf, an dem ein beweglicher Haltearm, genauer gesagt ein flexibler Gliederarm 14, befestigt ist, an dessen freien Ende ein Halteelement 16 zum Halten des Gewebes bzw. Organs vorgesehen ist. Die Vorrichtung 10 weist ferner eine Adaptionseinheit 18 auf, welche über mehrere Leitungen 20 mit dem Technikblock 12 in Verbindung steht. Eine Druckluftkartusche 22, die als externe Energiequelle dient und die das zum Betätigen des im Technikblock 12 integrierten Spannmechanismus für den Gliederarm 14 notwendige Arbeitsmedium liefert, kann an einem Druckluftanschluss 24 an der Adaptionseinheit 18 angeschlossen werden. Über eine Druckluftleitung 20a wird die Druckluft von der Adaptionseinheit 18 an den Technikblock 12 übertragen. Eine parallel dazu verlaufende Signalleitung 20b dient zur Steuerung der in der Adaptionseinheit 18 vorgesehenen (nicht gezeigten) Fluidsteuerelemente bzw. Ventile, welche vom Technikblock 12 bzw. vom Gliederarm 14 angesteuert werden können.

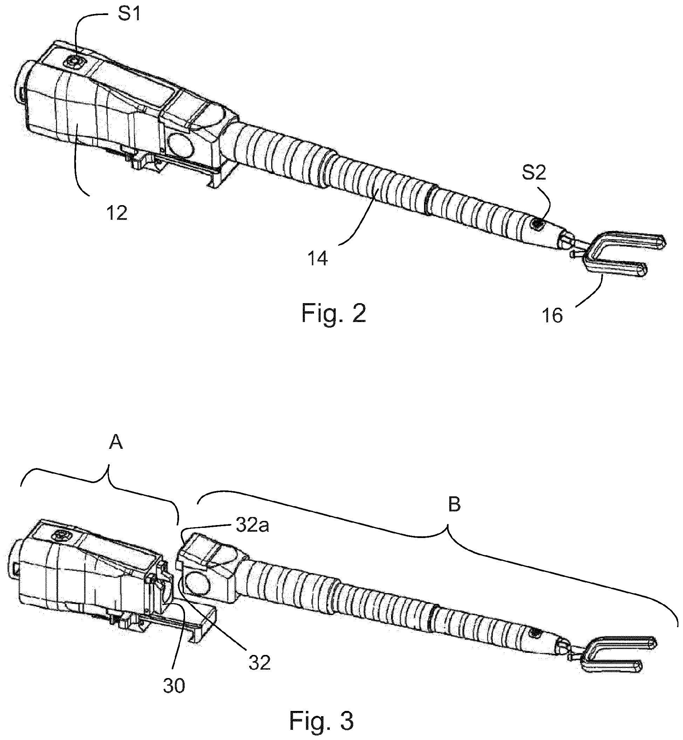

Die Fig. 2 zeigt lediglich den Technikblock 12, den Gliederarm 14 und das Halteelement 16 in einer vergrößerten Darstellung. Der Technikblock 12 enthält im Wesentlichen die gesamte Technik zum Spannen des Gliederarms 14 sowie zum Fixieren des Gesamtsystems an nicht gezeigten externen Halterungen, wie z. B. einen Sternumspreizer. Dabei bildet der Technikblock 12 ein wiederverwendbares Technikmodul A und der Gliederarm 14 samt Halteelement 16 ein für die Einmalverwendung konzipiertes Arbeitsmodul B (siehe Fig. 3). Die Technikmodule A und B repräsentieren beispielhaft zwei miteinander koppelbare Geräteeinheiten im Sinne der Ansprüche.

Aus der Fig. 2 ist ferner erkennbar, dass am Technikblock 12 ein erstes Betätigungselement S1 in Form eines elektrischen Tasters oder Schalters und am distalen Ende des Gliederarms 14 ein zweites Betätigungselement S2, ebenfalls in Form eines elektrischen Tasters oder Schalters, vorgesehen sind. Beide Betätigungselemente S1 und S2 dienen zur Steuerung des Energieflusses von der externen Energiequelle, d.h. zur Steuerung der Druckluft, die von der Druckluftkartusche 22 über die Druckluftleitung 20a und einen Druckluftanschluss 26 zum Technikblock 12 gelangt. Die Betätigungselemente S1, S2 bzw. die Ansteuerung der externen Energiequelle ist so ausgestaltet, dass im gekoppelten Zustand der beiden der Energiefluss solange freigegeben wird, solange die Betätigungselemente S1, S2 gedrückt bzw. betätigt werden.

Die Fig. 3 zeigt die von einander getrennten Module A und B und auch die dazugehörigen Kopplungsabschnitte 30 und 32 der beiden Module A und B.

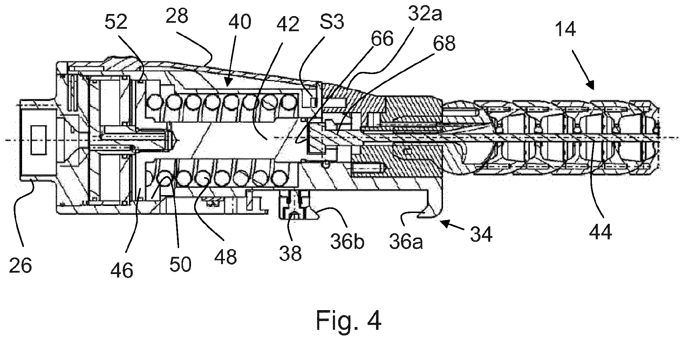

Die Fig. 4 zeigt eine Querschnittsansicht des Technikblocks 12 und eines Teils des damit gekoppelten Gliederarms 14. Der Technikblock 12 weist neben dem Kopplungsabschnitt 30 zum Ankoppeln des Arbeitsmoduls B einen Gehäuseabschnitt 28 und einen Befestigungsabschnitt 34 zum Befestigen des Technikblocks 12 an einer nicht gezeigten Halterung, wie einem Sternumspreizer. Der Gehäuseabschnitt 28 kann mit ergonomischen Griffmulden versehen werden, so dass die Montage bzw. Demontage des Arbeitsmoduls B vereinfacht werden kann.

Der Befestigungsabschnitt 34 weist zwei zueinander weitenverstellbare hinterschnittene Klemmbacken 36a und 36b auf, mit welchen der Technikblock 12 reib- und formschlüssig an einer entsprechenden Schiene oder ähnlichen Haltevorrichtung befestigt werden kann. Ein der beiden Klemmbacken 36b ist verstellbar und lässt sich ferner über eine Klemmschraube 38 fixieren.

Im Gehäuseabschnitt 28 befindet sich eine Zylinder-Kolben-Mechanik 40, welche einen wesentlichen Bestandteil des Spannmechanismus darstellt. Ein in dem Gehäuseabschnitt 28 in axialer Richtung, d.h. in Längsrichtung des Gliederarms 14, geführter Schieber oder Schlitten 42, der mit einem in dem Gliederarm 14 geführtes Zugseil 44 verbindbar ist, ist mit einem Ende eines Kolbens 46 der Zylinder-Kolben-Mechanik 40 verbunden, so dass eine Hubbewegung des Kolbens 46 zu einer translatorischen, axialen Verschiebung des Schlittens 42 führt.

Der Kolben 46 wird über eine Spiraldruckfeder 48 in eine Richtung vorgespannt, in welche der Kolben 46 über den Schlitten 42 das Zugseil 44 zum Technikblock 12 hin zieht und auf diese Weise den Gliederarm 14 bzw. dessen Gliederelemente verspannt und feststellt bzw. versteift. Aufgrund der Federvorspannung befindet sich der Gliederarm 14 im Ruhestand, d.h. ohne äußeren Eingriff, im festgestellten oder arretierten Zustand.

Um den an sich flexiblen Gliederarm 14 in beliebige Richtung biegen zu können, muss die Federvorspannung aufgehoben werden. Dies erfolgt mittels einer externen Energiequelle. Der Kolben 46 kann entgegen der Druckkraft der Spiraldruckfeder 48 mit Hilfe einer Druckluft, welche über den Druckluftanschluss 26 einem Zylinderraum 50 der Zylinder-Kolben-Mechanik 40 zugeführt wird, in die entgegengesetzte Richtung betätigt werden und dabei durch entsprechende Verschiebung des Schlittens 42 das Zugseil 44 freigeben oder entspannen und somit den festgestellten Gliederarm lösen.

Ein oder mehrere Kolbenringe 52 sorgen für die fluiddichte Trennung des mit Druckluft beaufschlagten Teils des Zylinderraums 50 von dem Teil des Zylinderraums 50, in dem sich die Spiraldruckfeder 48 befindet. Die Bewegung des Kolbens 46 bzw. des Schlittens 42 ist in beiden Richtungen jeweils durch Anschläge begrenzt, um definierte Verstellwege zum Lösen und Spannen vorzugeben.

Im Folgenden wird die Kopplung des Arbeitsmoduls B mit dem Technikmodul A detaillierter beschrieben.

Die Schnittstelle zwischen dem Arbeitsmodul B und dem Technikmodul B dient nicht nur zur rein mechanischen, sondern auch zur signaltechnischen und funktionalen Kopplung beider Einheiten, da zum einen die über das distale Betätigungselement S2 eingegebenen Steuersignale an den Technikblock 12 und von dort über die Steuersignalleitung 20b an die Adaptionseinheit 18 mit den entsprechenden Steuerorganen übertragen werden müssen und zum anderen das im Gliederarm 14 verlaufende Zugseil 44 mit der Zylinder-Kolben-Mechanik 40 gekoppelt werden muss.

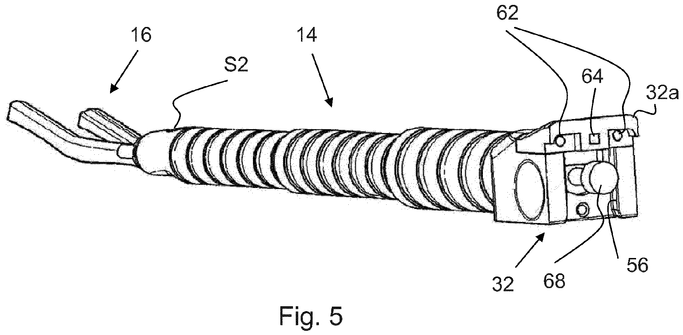

Zur mechanischen Kopplung des Moduls A mit dem Modul B weist der Kopplungsabschnitt 30 des Technikblocks 12, welcher sich unmittelbar oberhalb des Befestigungsabschnitts 34 befindet, eine sich vertikal erstreckende Schwalbenschwanzführung 54 auf, welche eine formschlüssige Verbindung mit einer komplementär ausgebildeten Führungsaufnahme 56 an einem Kopplungsabschnitt 32 des Arbeitsmodul B bildet (siehe Fig. 5). Hierfür wird der Kopplungsabschnitt 32 vertikal auf die Schwalbenschwanzführung 54 aufgeschoben, bis die beiden Kopplungsabschnitte 30 und 32 in flächige Anlage kommen. Zur lösbaren Arretierung beider Kopplungsabschnitte 30 und 32 ist am Kopplungsabschnitt 32 des Arbeitsmoduls B ein Verriegelungselement 58 in Form einer federvorgespannten Verriegelungsnase vorgesehen.

Der Kopplungsabschnitt 32 des Arbeitsmoduls B weist ferner einen zum Technikmodul A hin ragenden Vorsprungsabschnitt 32a auf, der den Kopplungsabschnitt 30 des Technikmoduls A von oben, d.h. in Zuführrichtung beim Koppeln der beiden Module A und B, abdeckt, so dass man mit der Hand oder den Fingern von oben nicht so leicht zwischen die beiden Kopplungsabschnitte 30 und 32 greifen kann.

Ferner sind an der Stirnseite des Kopplungsabschnitts 32, genauer gesagt an dem Vorsprungsabschnitt 32a, zwei elektrische Kontakte 62 vorgesehen, die mit entsprechenden Kontaktstellen 60 am Kopplungsabschnitt 30 des Technikblocks 12 in Kontakt kommen, wenn sich die beiden Module A und B in ihrer gekoppelten und verriegelten Arbeitsposition befinden. Die elektrischen Kontakte 62 stehen über (nicht dargestellte) elektrische Leitungen mit dem distalen Betätigungselement S2 in Verbindung.

Im Technikblock 12 sind ebenfalls (nicht dargestellte) Leitungen vorgesehen, welche die Kontaktstellen 60 mit der ausgehenden Steuerleitung 20b verbinden.

Am Kopplungsabschnitt 30 des Technikblocks 12 ist ferner ein Schaltelement insbesondere in Form eines Näherungsschalters S3 vorgesehen bzw. verbaut, der berührungslos aktiviert werden kann. Im beschriebenen Ausführungsbeispiel handelt es sich dabei um einen magnetischen Näherungsschalter, z.B. ein Reed-Relais oder ein Hallsensor, der durch einen kleinen Magneten 64 im proximalen Ende bzw. Kopplungsabschnitt 32 des Gliederarms 14 ausgelöst werden kann. Der Näherungsschalter S3 kann in den Technikblock 12 versenkt oder verdeckt eingebaut werden, um die Gefahr des Verschmutzens, Beschädigens und die Einflüsse beim Aufbereiten durch Sterilisation zu verringern. Je nach Stärke des Magneten 64 und Sensibilität des Näherungsschalters S3 kann sich auch eine gewisse Materialdicke (eines magnetisch nicht leitenden Materials) des Gehäuses 28 des Technikblocks 12 zwischen den beiden Bauteilen befinden. Durch entsprechende Auswahl bzw. Einstellung des Näherungsschalters S3 und des Magneten 64 kann ein Mindestabstand zwischen dem Technikblock 12 und dem Gliederarm 14, ab welchen der Näherungsschalter S3 ausgelöst wird, entsprechend eingestellt werden.

Aus der Fig. 5 ist ferner ein Endstück 68 des Zugseils 44 zu erkennen. Bei dem Endstück 68 handelt es sich um ein rotationssymmetrisches profiliertes Drehteil, welches passgenau in eine entsprechende Ausnehmung 66 im Schlitten 42 passt und auf diese Weise in axialer Richtung bzw. in Zugrichtung formschlüssig mit dem Schlitten 42 verbindbar ist. Das Endstück 68 am freien Ende des Zugseils 44 stellt ferner sicher, dass das durch den Kopplungsabschnitt 32 geführte Zugseil 44 nicht ausfädelt.

Die Ausnehmung 66 im Schlitten 42 stellt beispielhaft einen (ersten) Kopplungsabschnitt der ersten Funktionseinheit, d.h. des im Technikblock A vorgesehenen Teils des Spannmechanismus, und das Endstück 68 des Zugseils 44 stellt beispielhaft einen (zweiten) Kopplungsabschnitt der (zweiten) Funktionseinheit, d.h. des im Arbeitsmoduls B vorgesehen Teil des Spannmechanismus, dar.

Sowohl der Näherungsschalter S3 als auch der Magnet 64 befindet sich in unmittelbarer Nähe der zu koppelnden Abschnitte der beiden Funktionseinheiten, d.h. der Ausnehmung 66 im Schlitten 42 einerseits und des Endstücks 68 des Zugseils 44 andererseits.

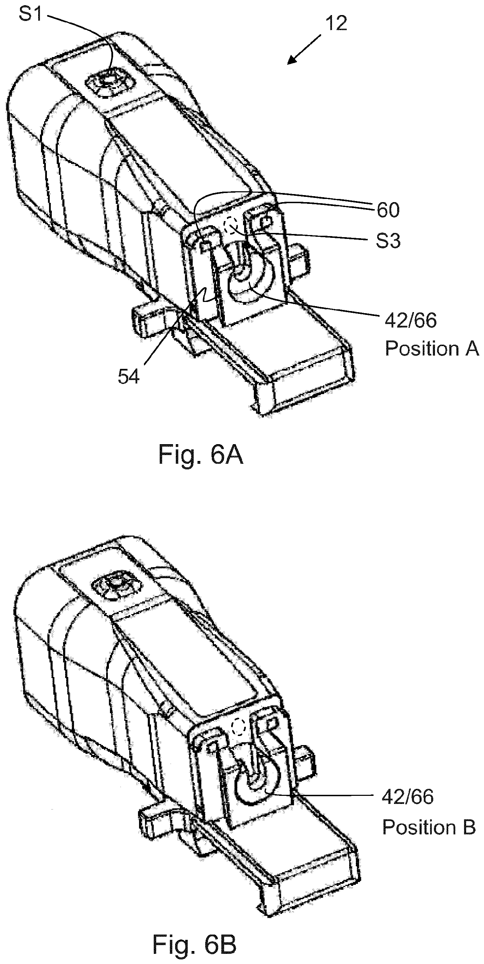

Die Fig. 6A zeigt eine perspektivische Ansicht des Technikblocks mit dem Schlitten 42 in seiner eingefahrenen Ruheposition (Position A) und Fig. 6B zeigt eine perspektivische Ansicht des Technikblocks mit dem Schlitten 42 in seiner ausgefahrenen Kopplungsposition (Position B). In der eingefahrenen Ruheposition (Position A) ist der Schlitten 42 samt Ausnehmung 66 soweit in das Gehäuse 28 eingefahren, dass das Endstücks 68 des Zugseils 44 nicht eingelegt werden kann. In dieser Position stellt der Schlitten 42 keine Gefahr für den Anwender dar.

Im Folgenden wird die Kopplung des Schlittens 42 (erste Funktionseinheit) mit dem Zugseil 44 (zweite Funktionseinheit) detailliert beschrieben:

Das Endstück 68 des Zugseils 44 kann in die Ausnehmung 66 von oben eingelegt werden, wenn sich der Schlitten 42 in der voll ausgefahrenen Position bzw. einer Kopplungsposition befindet. Da der Schlitten 42 im Ruhezustand aufgrund der Vorspannung der Feder 48 eingefahren ist, muss zum Einlegen des Endstücks 68 in die Ausnehmung 66 der Schlitten 42 über die Zylinder-Kolben-Mechanik 40 ausgefahren werden.

Solange der Schlitten 42 aus dem Gehäuse herausgefahren (Kopplungsposition B) und der Gliederarm 14 noch nicht adaptiert ist, stellt die offene Ausnehmung 66 im Schlitten 42 in Verbindung mit der starken Federkraft Feder 48 eine erhebliche Gefahrenquelle für den Anwender dar.

Wenn sich, wie oben beschrieben, ein Magnet 64 im proximalen Ende des Gliederarms 14 ausreichend nah am Näherungsschalter S3 des Technikblocks 12 befindet, gibt die Schaltung das Ausfahren des Schlittens 42 frei. Die Stärke des Magneten 64 und die Empfindlichkeit des Näherungsschalters S3 sind dabei so abgestimmt, dass der Schlitten 42 erst ausfahren kann, wenn das proximale Ende des Gliederarms 14, genauer gesagt der Vorsprungsabschnitt 32a, die Ausnehmung 66 im Schlitten 42 ausreichend verdeckt, so dass es dem Anwender nicht möglich ist, beispielsweise mit einem Finger dazwischen zu fassen.

Ist der Gliederarm 14 vollständig adaptiert, liegen die beiden Kopplungsabschnitte 30 und 32 flächig aneinander und wird der elektrische Kontakt zwischen den Kontaktstellen 60 am Technikblock und den Kontakten/Gegenelementen 62 des Gliederarmes 14 geschlossen. Im adaptierten und unbetätigten Grundzustand sind Kontaktstellen 60 und die Gegenelemente 62 durch den Öffner S2 kurzgeschlossen. So ist es möglich, zwischen den Zuständen "im Adaptionsvorgang befindlich" und "betriebsbereit" zu unterscheiden.

Um das Schließen des Kontakts zu bewerkstelligen, muss es sich beim Tast-Schalter S2 des Haltearmes S2 um einen elektrischen Öffner-Kontakt handeln. Durch dieses Signal wird der Schlitten 42 wieder zurückgefahren und spannt dabei das Zugseil 44 im Gliederarm 14. Nun kann mit den beiden Betätigungselementen S1 und S2 normal gearbeitet werden und durch Drücken eines der Betätigungselemente S1 oder S2 der Spannmechanismus und somit die Arretierung des Gliederarms 14 gelöst und durch Loslassen bzw. Nichtbetätigung der Betätigungselemente S1 oder S2 der Gliederarm 14 wieder arretiert werden.

Zum Demontieren des Gliederarmes 14 wird das Betätigungselement S1 auf der Technikeinheit S1 gedrückt und der Schlitten 42 fährt aus. Sobald der Gliederarm 14 ein Stück weit angehoben wird, wird der elektrische Kontakt zwischen den Kontaktstellen 60, 62 geöffnet und das Signal, welches den Schlitten 42 ausfahren lässt, wird von der Schaltung beibehalten.

Der Anwender kann nun das Betätigungselement S1 loslassen und hat beide Hände für die weitere Demontage zur Verfügung. Ist der Anschluss des Gliederarms 14 vollständig aus dem Schlitten 42 herausgezogen und der Gliederarm 14 von der Technikeinheit 12 entfernt, so wird, wenn sich der Magnet 64 entsprechend weit (um mindestens den gewünschten und eingestellten Mindestabstand) entfernt hat, das Signal des Näherungsschalters S3 unterbrochen und der Schlitten 42 fährt wieder in seine Ruheposition zurück.

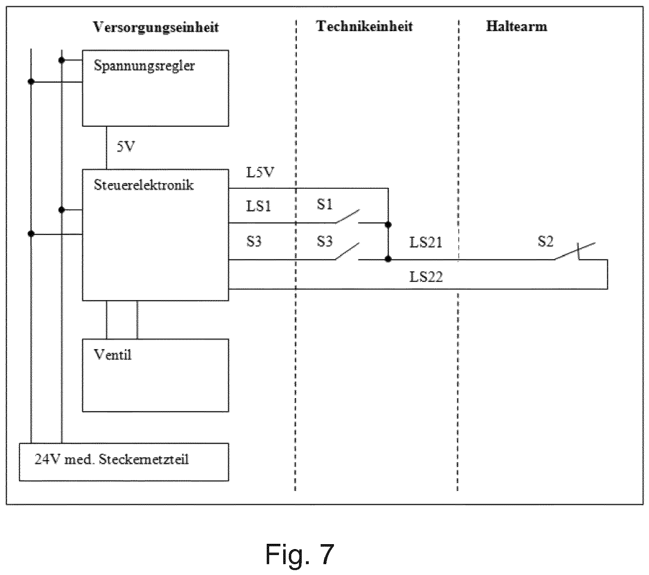

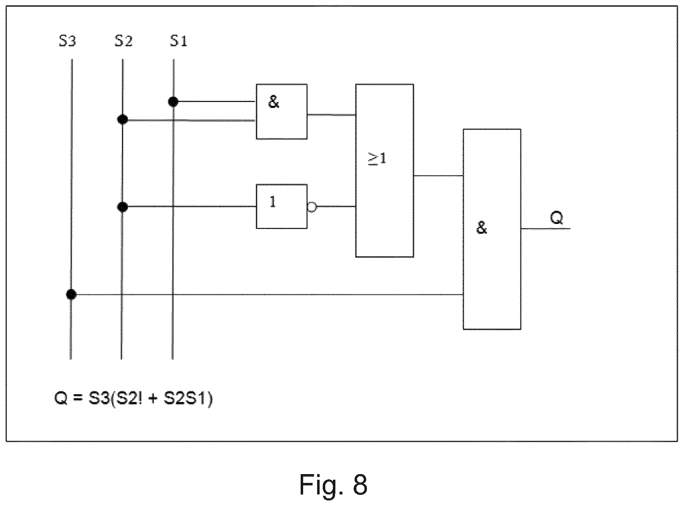

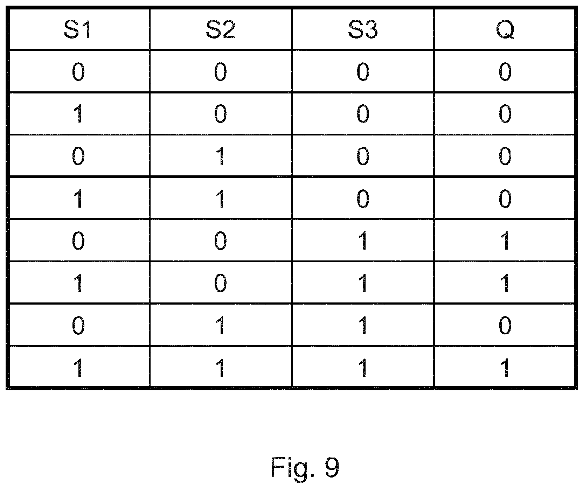

Im Folgenden werden die Funktionen bzw. die Schaltbefehle der Betätigungselemente bzw. Schalter mit Bezug auf die Fign. 7 bis 9 detailliert beschrieben, wobei die Fig. 7 ein elektrisches Schaltbild, Fig. 8 eine Schaltlogik und Fig. 9 eine Logiktabelle zeigen.

Im Technikblock (erste Geräteinheit) hat das Betätigungselement bzw. der Schließer S1 die Funktion, den Gliederarm freizugeben. Der Näherungsschalter S3 dient zur Erkennung eines Adaptions- oder Kopplungsvorgangs des Gliederarms 14. Der Näherungsschalter S3 ist so konfiguriert, dass er beim Kopplungsvorgang früh schaltet, so dass das Seilende 68 in den Schlitten 42 eingehängt werden kann, und beim Entkopplungsvorgang spät schaltet, um sicher zu gehen, dass das Seilende 68 vollständig aus dem Schlitten 42 herausgenommen worden ist.

Das am Gliederarm 14 vorgesehene Betätigungselement bzw. der Öffner S2 hat ebenfalls die Funktion, den Gliederarm freizugeben.

Zum Koppeln der beiden Funktionseinheiten Schlitten 42 und Seil 44 ist zunächst der Näherungsschalter S3 offen. Weder das Betätigungselement S1 noch das Betätigungselement S2 muss betätigt sein oder werden. Schließt der Näherungsschalter S3 bedingt durch die Annäherung des am Haltearm 14 vorgesehen Magneten 64, fährt der Schlitten 42 in die Kopplungsposition heraus und das Seilende 68 kann eingehängt werden. Wenn die beiden Module A und B vollständig gekoppelt sind, wird ein elektrischer Kontakt zwischen den Kontakten 60 und 62, d.h. zwischen beiden Modulen A und B, hergestellt und der Öffner S2 geschlossen, woraufhin der Schlitten 42 wieder in seine Ruheposition einfährt und das medizinische Instrument 10 betriebsbereit ist.

Beim Entkoppeln oder Entfernen des Arbeitsmoduls B bzw. des Gliederarms 14 ist zunächst der Näherungsschalter S3 geschlossen und der Schlitten 42 betriebsbereit. Wird einer der beiden Betätigungselemente, d.h. entweder der Schließer S1 oder der Öffner S2 betätigt, fährt der Schlitten 42 aus. Wenn der Gliederarm 14 vom Technikblock 12 gelöst wird, wird der elektrische Kontakt zwischen den Kontakten 60 und 62 unterbrochen und S2 geöffnet. Dadurch verbleibt der Schlitten 42 in seiner ausgefahrenen Position bzw. Kopplungs-/Entkopplungsposition, ohne eines der beiden Betätigungselemente S1 oder S2 weiterhin betätigen zu müssen. In dieser Kopplungsposition wird das Seilende 68 freigegeben und das Arbeitsmodul B kann vollständig entfernt werden. Ab einem bestimmten Abstand (Mindestabstand) zwischen Magnet 64 und Näherungsschalter S3 öffnet der Schließer S3 und der Schlitten 42 fährt aufgrund der Federvorspannung in den Technikblock 12 ein.

In der in der Fig. 9 dargestellten Logiktabelle repräsentiert Q die Stellung des Schlittens 42, wobei "1" die ausgefahrene Position bzw. Kopplungsposition und "0" die eingefahrene Position bzw. Ruheposition bedeutet. Ist der Näherungsschalter S3 offen, d.h. der Abstand zwischen beiden Geräteinheiten 12, 14 ist größer oder gleich dem Mindestabstand, kann der Schlitten 42 selbst bei Betätigen (Schließen) des Schalters S1 nicht in die Kopplungsposition gebracht werden. Ist der Näherungsschalter S3 geschlossen, d.h. der Abstand zwischen beiden Geräteinheiten 12, 14 ist kleiner als der Mindestabstand, und ist der Gliederarm noch nicht vollständig (elektrisch) gekoppelt, fährt der Schlitten 42 auch ohne Betätigung des Schalters S1 in die Kopplungsposition. Ist der Gliederarm mechanisch und elektrisch gekoppelt, ist Schalter S2 geschlossen und der Schlitten 42 fährt bedingt durch die Federvorspannung automatisch zurück in die Ruheposition.

Im mechanisch und elektrisch gekoppelten und somit betriebsbereiten Zustand lässt sich der Schlitten durch Betätigen (Schließen) des Schalters S1 oder durch Betätigen (Öffnen) des Schalters S2 ausfahren und dadurch der Gliederarm 14 lösen bzw. durch Loslassen des jeweiligen Schalters mittels der Federvorspannung der Gliederarm 14 wieder spannen und arretieren.

Zum Entkoppeln kann S1 betätigt (geschlossen) oder S2 betätigt (geöffnet) werden, um den Schlitten 42 in die Kopplungsposition zu bringen. Wird der Gliederarm 14 von dem Technikblock 12 getrennt, löst sich zunächst der elektrische Kontakt zwischen dem Gliederarm 14 und dem Technikblock 12, was einem dauerhaften Betätigen (Öffnen) des Schalters S2 gleichkommt. Nach Trennen des elektrischen Kontakts kann S1 oder S2 losgelassen werden und der Schlitten 46 verbleibt in der Kopplungsposition, solange der Näherungsschalter S3 (noch) geschlossen ist. Wird der Abstand zwischen Magnet 64 und Näherungsschalter S3 zu groß, öffnet der Näherungsschalter S3 und der Schlitten 46 fährt bedingt durch die Federvorspannung automatisch zurück in die Ruheposition.

Die vorliegende Erfindung beschreibt somit ein elektrisches Schaltkonzept an einem zweiteiligen chirurgischen Gerät, das kraftbetätigt arbeitet und elektrisch gesteuert wird, wobei die Schalt-Logik die Risiken des Geräts, die insbesondere vor oder bei dem Adaptieren des zweiten Geräteteils vorliegen, minimiert.

Dadurch ist der Montage- und Demontagevorgang einerseits sehr sicher, da der Schlitten 42 nur ausfahren kann, wenn er durch das hintere bzw. proximale Ende des Gliederarmes 14 ausreichend abgedeckt wird und somit keine Gefahrenquelle mehr darstellt. Andererseits sind die Vorgänge sehr komfortabel, da der Anwender zum Montieren der beiden Teile gar kein Betätigungselement und zur Demontage ein Betätigungselement nur kurz aktivieren muss.

Dadurch, dass die Schaltlogik in Verbindung mit dem Näherungsschalter automatisch für die entsprechenden Bewegungen sorgt, kann diese Sicherheitseinrichtung auch nicht vom Anwender nicht beachtet und vergessen werden.

Die vorliegende Erfindung ist jedoch nicht auf die zuvor detailliert beschriebenen Ausführungsformen beschränkt, sondern kann innerhalb des Schutzbereichs der beigefügten Ansprüche variiert werden. Im Folgenden sind einige solcher Variationsmöglichkeiten aufgeführt.

Im vorliegenden Ausführungsbeispiel wird der Technikblock wie eingangs beschrieben von einer weiteren Einheit (Steckernetzteil) mit Energie versorgt. Alternativ kann der Technikblock auch alle Energien selber beinhalten oder bereitstellen. Die Steuerelektronik ist weiterhin aufgrund der Sterilisationsproblematik (hohe Temperatur, Wasserdampf) ausgelagert. Bei entsprechender Anpassung könnte sie ebenfalls in der Technikeinheit untergebracht sein.

In weiteren Ausführungsbeispielen kann die Schaltlogik durch den Einbau mehrerer Betätigungsschalter noch geringfügig verändert werden. Beim beschriebenen Ausführungsbeispiel mit zwei Betätigungselementen sind aber mindestens drei Schalter notwendig. Das Prinzip dieser Sicherheitsschaltung kann ebenso auf mehrteilige Geräte angewendet werden.

Unterschiedliche Ausführungsbeispiele ergeben auch bei der Wahl des Sensors S3. Neben dem oben genannten magnetischen Näherungsschalter sind Ausführungsbeispiele in Form von optischen Sensoren, kapazitiven Sensoren, induktiven Sensoren, mechanischen Schaltelementen, Kontaktstellen, welche vom Haltearm überbrückt werden, elektromagnetischen Näherungsschaltern, oder Schaltelementen, die mittels einer Lichtschranke oder Ultraschall betrieben werden, denkbar.

Darüber hinaus sind modifizierte Ausführungsbeispiele denkbar, in welchen eines der Betätigungselemente S1 und S2 entfällt.

Die Steuerungselektronik kann mittels diskreter Logikgatter-Bausteine, Mikrocomputer oder Schaltungen aus einzelnen Transistoren und Dioden aufgebaut werden. Mittels entsprechenden Zusatzkomponenten oder speziellen Programmen im Mikrocomputer können zudem Zeitkonstanten implementiert werden, welche das Prellen der mechanischen Schaltelemente filtern. Solche Zeitkonstanten können auch dazu verwendet werden, Störungen aus Übertragungsleitungen zu filtern (diese können beispielsweise durch HF-Chirurgiegeräte einkoppeln).

Es ist ebenfalls möglich die Auswirkungen eines "Zittern" des Signals vom Magnetsensor mittels der Implementierung von Zeitkonstanten zu eliminieren. Ein solches Zittern kann insbesondere dann auftreten, wenn der Magnet in einem Abstand zum Sensor bewegt wird, der sich nahe an der Schaltschwelle befindet. Über die Implementierung von Zeitkonstanten kann darüber hinaus erreicht werden, dass Schaltzustände für eine Mindest- oder Maximaldauer gehalten werden.

### Bezugszeichenliste

- 10: Vorrichtung
- 12: Grundkörper/Technikblock
- 14: Gliederarm
- 16: Halteelement
- 18: Adaptionseinheit
- 20: Leitungen
- 20a: Druckluftleitung
- 20b: Steuerleitung
- 22: Druckluftkartusche
- 24: Druckluftanschluss
- 26: Druckluftanschluss
- 28: Gehäuseabschnitt
- 30: Kopplungsabschnitt
- 32: Kopplungsabschnitt
- 32a: Vorsprungsabschnitt
- 34: Befestigungsabschnitt
- 36a, 36b: Klemmbacken
- 38: Klemmschraube
- 40: Zylinder-Kolben-Mechanik
- 42: Schlitten
- 44: Zugseil
- 46: Kolben
- 48: Spiraldruckfeder
- 50: Zylinderraum
- 52: Kolbenring
- 54: Schwalbenschwanzführung
- 56: Führungsaufnahme
- 58: Verriegelungsnase
- 60: elektrische Kontakte
- 62: elektrische Gegenkontakte
- 64: Magnet
- 66: Ausnehmung
- 68: Endstück

- A: Technikmodul
- B: Arbeitsmodul
- S1: erstes Betätigungselement
- S2: zweites Betätigungselement
- S3: Näherungsschalter

## Patentansprüche

1. Medizintechnisches Gerät (10) mit zwei miteinander mechanisch koppelbaren Geräteeinheiten (12, 14), wobei
die beiden Geräteeinheiten (12, 14) zwei miteinander mechanisch koppelbare Funktionseinheiten (42, 44) aufweisen, um im mechanisch gekoppelten Zustand mit der ersten Funktionseinheit (42) die zweite Funktionseinheit (44) betätigen zu können; und
die erste Funktionseinheit (42) elektrisch oder elektronisch ansteuerbar ist und einen mechanischen Kopplungsabschnitt (66) aufweist, der zum Koppeln mit einem entsprechenden mechanischen Kopplungsabschnitt (68) der zweiten Funktionseinheit (44) in eine Kopplungsposition (B) bringbar ist,
**dadurch gekennzeichnet, dass**
zumindest an einer Geräteeinheit (12, 14) Mittel (S3, 64) zum Erfassen einer Relativlage oder eines Abstands der beiden Geräteeinheiten (12, 14) zueinander vorgesehen sind; und
die elektrische oder elektronische Ansteuerung der ersten Funktionseinheit (42) derart konfiguriert ist, dass der mechanische Kopplungsabschnitt (66) der ersten Funktionseinheit (42) nur dann in die Kopplungsposition (B) bringbar ist, wenn sich die beiden Geräteinheiten (12, 14) in einer bestimmten Relativlage zueinander befinden oder einen vorbestimmten Mindestabstand zueinander unterschreiten.

2. Medizintechnisches Gerät (10) nach Anspruch 1, **dadurch gekennzeichnet, dass** die Relativlage oder der Mindestabstand so gewählt ist, dass ein Benutzer mit seiner Hand oder seinem Finger nicht zwischen beide Geräteinheiten (12, 14) greifen kann oder die beiden Geräteeinheiten (12, 14) die beiden Kopplungsabschnitte (66, 68) der Funktionseinheiten (42, 44) derart verdecken, dass ein Benutzer mit seiner Hand oder seinem Finger diese nicht erreichen kann.

3. Medizintechnisches Gerät (10) nach einem der vorhergehenden Ansprüche, **dadurch gekennzeichnet, dass** die Mittel zum Erfassen einer Relativlage oder eines Abstands der beiden Geräteeinheiten (12, 14) ein am oder zumindest in der Nähe des Kopplungsabschnitts (66) der ersten Funktionseinheit (42) vorgesehener, insbesondere magnetischer, Nährungsschalter oder -sensor (S3) ist, der durch am oder zumindest in der Nähe des Kopplungsabschnitts (68) der zweiten Funktionseinheit (44) vorgesehene Mittel, insbesondere Magneten (64), auslösbar ist, wenn der erste Kopplungsabschnitt (66) in die Nähe des zweiten Kopplungsabschnitts (68) gebracht wird und dabei der Mindestabstand unterschritten wird.

4. Medizintechnisches Gerät (10) nach Anspruch 3, **dadurch gekennzeichnet, dass** der Nährungsschalter oder -sensor (S3) in der Geräteinheit (12) der ersten Funktionseinheit (42) versenkt oder verdeckt eingebaut ist.

5. Medizintechnisches Gerät (10) nach einem der vorhergehenden Ansprüche, **dadurch gekennzeichnet, dass** der Kopplungsabschnitt (66) der ersten Funktionseinheit (42) in eine vorbestimmte Position (A), insbesondere Ruheposition (A), federvorgespannt ist und mittels Fremdenergie, insbesondere hydraulischem oder pneumatischen Druck, in die Kopplungsposition (B) bringbar ist.

6. Medizintechnisches Gerät (10) nach einem der vorhergehenden Ansprüche, **dadurch gekennzeichnet, dass** die Geräteeinheiten (12, 14), wenn sie miteinander mechanisch gekoppelt sind, auch elektrisch gekoppelt sind, wobei
beim Koppeln der beiden Geräteeinheiten (12, 14) die elektrische Kopplung erst bei vollständiger mechanische Kopplung erfolgt; und
beim Entkoppeln der beiden Geräteeinheiten (12, 14) die elektrische Kopplung vor der mechanischen Kopplung gelöst wird.

7. Medizintechnisches Gerät (10) nach Anspruch 6, **dadurch gekennzeichnet, dass** der Kopplungsabschnitt (66) der ersten Funktionseinheit (42) nach dem elektrischen Koppeln der beiden Geräteeinheiten (12, 14) automatisch aus der Kopplungsposition (B) in die vorbestimmte Position (A) gebracht wird.

8. Medizintechnisches Gerät (10) nach Anspruch 6 oder 7, **dadurch gekennzeichnet, dass** der Kopplungsabschnitt (66) der ersten Funktionseinheit (42) nach dem elektrischen Entkoppeln der beiden Geräteeinheiten (12, 14) automatisch in der Kopplungsposition (B) befindet und solange in der Kopplungsposition (B) gehalten wird, bis der Abstand zwischen beiden Geräteeinheiten (12, 14) größer oder gleich der Mindestabstand ist, und nach Überschreiten des Mindestabstands automatisch in die vorbestimmte Position (A) gebracht wird.

9. Medizintechnisches Gerät (10) nach einem der Ansprüche 5 bis 8, **dadurch gekennzeichnet, dass**
im gekoppelten Zustand der beiden Geräteeinheiten (12, 14) die erste Funktionseinheit (42) und mittelbar die zweite Funktionseinheit (44) durch Betätigen eines ersten Betätigungselements (S1), das an der ersten Geräteeinheit (12) vorgesehen ist, elektrisch oder elektronisch ansteuerbar sind; und
im entkoppelten Zustand der beiden Geräteeinheiten (12, 14) der Kopplungsabschnitt (66) der ersten Funktionseinheit (42) mittels der Fremdenergie automatisch in die Kopplungsposition (B) gebracht wird, wenn sich die beiden Geräteinheiten (12, 14) in der bestimmten Relativlage zueinander befinden oder den vorbestimmten Mindestabstand zueinander unterschritten haben.

10. Medizintechnisches Gerät (10) nach einem der Ansprüche 5 bis 8, **dadurch gekennzeichnet, dass**
im gekoppelten Zustand der beiden Geräteeinheiten (12, 14) die erste Funktionseinheit (42) und mittelbar die zweite Funktionseinheit (44) durch Betätigen eines zweiten Betätigungselements (S2), das an der zweiten Funktionseinheit (44) vorgesehen ist, elektrisch oder elektronisch ansteuerbar sind; und
das zweite Betätigungselement (S2) derart ausgebildet ist, insbesondere als elektrischer Öffner-Kontakt ausgebildet ist, so dass beim elektrischen Koppeln der beiden Geräteeinheiten (12, 14) die Energiezufuhr für die Ansteuerung der ersten Funktionseinheit (12) unterbrochen wird.

11. Medizintechnisches Gerät (10) nach einem der vorhergehenden Ansprüche, **dadurch gekennzeichnet, dass**
der Mindestabstand kleiner oder gleich 40 mm beträgt, insbesondere kleiner oder gleich 20 mm, besonders bevorzugt kleiner oder gleich 15 mm.

12. Verfahren zum mechanischen Koppeln von zwei Funktionseinheiten (42, 44) eines zwei- oder mehrteiligen medizintechnisches Geräts (10), wobei
das medizintechnische Gerät (10) zwei miteinander mechanisch koppelbaren Geräteeinheiten (12, 14) aufweist,
die beiden Geräteeinheiten (12, 14) zwei miteinander mechanisch koppelbare Funktionseinheiten (42, 44) aufweisen, um im mechanisch gekoppelten Zustand mit der ersten Funktionseinheit (42) die zweite Funktionseinheit (44) betätigen zu können; und
die erste Funktionseinheit (42) elektrisch oder elektronisch ansteuerbar ist und einen mechanischen Kopplungsabschnitt (66) aufweist, der zum Koppeln mit einem entsprechenden mechanischen Kopplungsabschnitt (68) der zweiten Funktionseinheit (44) in eine Kopplungsposition (B) bringbar ist,
**gekennzeichnet durch** die Schritte:
Erfassen einer Relativlage oder eines Abstands der beiden Geräteeinheiten (12, 14) zueinander; und
Konfigurieren der elektrischen oder elektronischen Ansteuerung der ersten Funktionseinheit (42) derart, dass der mechanische Kopplungsabschnitt (66) der ersten Funktionseinheit (42) nur dann in die Kopplungsposition gebracht wird, wenn sich die beiden Geräteinheiten (12, 14) in einer bestimmten Relativlage zueinander befinden oder einen vorbestimmten Mindestabstand zueinander unterschreiten.

## Claims

1. A medical device (10) comprising two device units (12, 14) that can be mechanically coupled to each other, wherein
the two device units (12, 14) have two functional units (42, 44) that can be mechanically coupled to each other in order to be able to actuate the second functional unit (44) by means of the first functional unit (42) in the mechanically coupled state; and
the first functional unit (42) can be controlled electrically or electronically and has a mechanical coupling segment (66) which can be brought into a coupling position (B) in order to couple with a corresponding mechanical coupling segment (68) of the second functional unit (44),
**characterized in that**
means (S3, 64) for detecting a relative position of the two device units (12, 14) with respect to each other or a distance of the two device units from each other are provided at least on one device unit (12, 14); and
the electrical or electronic control of the first functional unit (42) is configured in such a way that the mechanical coupling segment (66) of the first functional unit (42) can be brought into the coupling position (B) only if the two device units (12, 14) are in a certain relative position with respect to each other or fall bellow a predetermined minimum distance from each other.

2. The medical device (10) according to claim 1, **characterized in that** the relative position or the minimum distance is selected such that a user cannot put his hand or finger between both device units (12, 14) or the two device units (12, 14) cover the two coupling segments (66, 68) of the functional units (42, 44) in such a way that a user cannot reach them with his hand or finger.

3. The medical device (10) according to any of the preceding claims, **characterized in that** the means for detecting a relative position or a distance between the two device units (12, 14) is in particular a magnetic proximity switch or proximity sensor (S3) provided on or at least near the coupling segment (66) of the first functional unit (42), said switch or proximity sensor being able to be triggered by appliances, in particular by magnets (64), which are provided on or at least near the coupling segment (68) of the second functional unit (44), said triggering being effected if the first coupling segment (66) is brought near the second coupling segment (68) while falling below the minimum distance.

4. The medical device (10) according to claim 3, **characterized in that** the proximity switch or proximity sensor (S3) is installed in the device unit (12) of the first functional unit (42) so as to be flush-mounted or concealed.

5. The medical device (10) according to any of the preceding claims, **characterized in that** the coupling segment (66) of the first functional unit (42) is spring-biased into a predetermined position (A), especially resting position (A), and can be brought to the coupling position (B) by means of external power, in particular by a hydraulic or pneumatic pressure.

6. The medical device (10) according to any of the preceding claims, **characterized in that** the device units (12, 14) are also electrically coupled if they are mechanically coupled to each other, wherein
during coupling of the two device units (12, 14), the electrical coupling is not effected until the mechanical coupling is completed; and
during decoupling of the two device units (12, 14), the electrical coupling is released before the mechanical coupling.

7. The medical device (10) according to claim 6, **characterized in that** the coupling segment (66) of the first functional unit (42) is automatically shifted from the coupling position (B) to the predetermined position (A) after the electrical coupling process of the two device units (12, 14).

8. The medical device (10) according to claim 6 or 7, **characterized in that** the coupling segment (66) of the first functional unit (42) is automatically in the coupling position (B) after the electrical decoupling of the two device units (12, 14) and is kept in the coupling position (B) until the distance between two device units (12, 14) is larger than or equal to the minimum distance, and is automatically brought into the predetermined position (A) after exceeding the minimum distance.

9. The medical device (10) according to any of the claims 5 to 8, **characterized in that**
in the coupled state of the two device units (12, 14), the first functional unit (42) and indirectly the second functional unit (44) can be electrically or electronically actuated by operating a first actuation element (S1) provided on the first device unit (12); and
in the decoupled state of the two device units (12, 14), the coupling segment (66) of the first functional unit (42) is automatically brought into the coupling position (B) by means of the external power, if the two device units (12, 14) are in the specific relative position with respect to each other or have fallen below the predetermined minimum distance with respect to each other.

10. The medical device (10) according to any of the claims 5 to 8, **characterized in that**
in the coupled state of the two device units (12, 14), the first functional unit (42) and indirectly the second functional unit (44) can be electrically or electronically actuated by operating a second actuation element (S2) provided on the second functional unit (44); and
the second actuation element (S2) is configured in particular as an electrical break contact and such that the supply of energy for the activation of the first functional unit (12) is interrupted during the electrical coupling of the two device units (12, 14).

11. The medical device (10) according to any of the preceding claims, **characterized in that**
the minimum distance is equal to or smaller than 40 mm, in particular equal to or smaller than 20 mm, particularly preferred equal to or smaller than 15 mm.

12. A method of mechanically coupling two functional units (42, 44) of a two-piece or multi-piece medical device (10), wherein
the medical device (10) comprises two device units (12, 14) that can be mechanically coupled to each other,
the two device units (12, 14) have two functional units (42, 44) that can be mechanically coupled to each other in order to be able to actuate the second functional unit (44) by means of the first functional unit (42) in the mechanically coupled state; and
the first functional unit (42) can be controlled electrically or electronically and has a mechanical coupling segment (66) which can be brought into a coupling position (B) in order to couple with a corresponding mechanical coupling segment (68) of the second functional unit (44),
**characterized by** the steps:
detecting a relative position or a distance of the two device units (12, 14) relative to each other; and
configuring the electrical or electronic control of the first functional unit (42) in such a manner that the mechanical coupling segment (66) of the first functional unit (42) is brought into the coupling position only if the two device units (12, 14) are in a specific relative position with respect to each other or fall below a predetermined minimum distance with respect to each other.

## Revendications

1. Appareil médical (10) avec deux unités d'appareil (12, 14) pouvant être couplées mécaniquement l'une à l'autre, dans lequel
les deux unités d'appareil (12, 14) présentent deux unités fonctionnelles (42, 44) pouvant être couplées mécaniquement l'une à l'autre afin de pouvoir actionner, à l'état couplé mécaniquement avec la première unité fonctionnelle (42), la seconde unité fonctionnelle (44) ; et
la première unité fonctionnelle (42) peut être commandée électriquement ou électroniquement et présente une section de couplage mécanique (66) qui peut être amenée dans une position de couplage (B) pour le couplage avec une section de coupage (68) mécanique correspondante de la seconde unité fonctionnelle (44),
**caractérisé en ce que**
au moins sur une unité d'appareil (12, 14), des moyens (S3, 64) sont prévus pour la détection d'une position relative ou d'une distance des deux unités d'appareil (12, 14) l'une par rapport à l'autre ; et
la commande électrique ou électronique de la première unité fonctionnelle (42) est configurée de telle manière que la section de couplage mécanique (66) de la première unité fonctionnelle (42) ne puisse être amenée dans la position de couplage (B) que lorsque les deux unités d'appareil (12, 14) se trouvent dans une position relative déterminée l'une par rapport à l'autre ou n'atteignent pas une distance minimale prédéterminée l'une de l'autre.

2. Appareil médical (10) selon la revendication 1, **caractérisé en ce que** la position relative ou la distance minimale est choisie de sorte qu'un utilisateur ne puisse saisir avec sa main ou son doigt entre les deux unités d'appareil (12, 14) ou les deux unités d'appareil (12, 14) cachent les deux sections de couplage (66, 68) des unités fonctionnelles (42, 44) de telle manière qu'un utilisateur ne puisse les atteindre avec sa main ou son doigt.

3. Appareil médical (10) selon l'une quelconque des revendications précédentes, **caractérisé en ce que** les moyens de détection d'une position relative ou d'une distance des deux unités d'appareil (12, 14) est un commutateur ou capteur de proximité (S3), en particulier magnétique, prévu sur ou au moins à proximité de la section de couplage (66) de la première unité fonctionnelle (42), qui peut être déclenché par des moyens prévus sur ou au moins à proximité de la section de couplage (68) de la seconde unité fonctionnelle (44), en particulier des aimants (64), lorsque la première section de couplage (66) est amenée à proximité de la seconde section de couplage (68) et la distance minimale n'est pas atteinte.

4. Appareil médical (10) selon la revendication 3, **caractérisé en ce que** le commutateur ou capteur de proximité (S3) est intégré en étant encastré ou caché dans l'unité d'appareil (12) de la première unité fonctionnelle (42).

5. Appareil médical (10) selon l'une quelconque des revendications précédentes, **caractérisé en ce que** la section de couplage (66) de la première unité fonctionnelle (42) est précontrainte par ressort dans une position prédéterminée (A), en particulier une position de repos (A), et peut être amenée dans la position de couplage (B) au moyen d'une énergie extérieure, en particulier une pression hydraulique ou pneumatique.

6. Appareil médical (10) selon l'une quelconque des revendications précédentes, **caractérisé en ce que** les unités d'appareil (12, 14), lorsqu'elles sont couplées mécaniquement ensemble, sont aussi couplées électriquement, dans lequel
lors du couplage des deux unités d'appareil (12, 14), le couplage électrique n'est effectué qu'en cas de couplage mécanique complet ; et
lors du découplage des deux unités d'appareil (12, 14), le couplage électrique est détaché avant le couplage mécanique.

7. Appareil médical (10) selon la revendication 6, **caractérisé en ce que** la section de couplage (66) de la première unité fonctionnelle (42) est automatiquement amenée après le couplage électrique des deux unités d'appareil (12, 14) de la position de couplage (B) à la position prédéterminée (A).

8. Appareil médical (10) selon la revendication 6 ou 7, **caractérisé en ce que** la section de couplage (66) de la première unité fonctionnelle (42) se trouve automatiquement dans la position de couplage (B) après le découplage électrique des deux unités d'appareil (12, 14) et est maintenue dans la position de couplage (B) jusqu'à ce que la distance entre les deux unités d'appareil (12, 14) soit plus grande ou identique à la distance minimale et est amenée automatiquement dans la position prédéterminée (A) après le dépassement de la distance minimale.

9. Appareil médical (10) selon l'une des revendications 5 à 8, **caractérisé en ce que**
dans l'état couplé des deux unités d'appareil (12, 14), la première unité fonctionnelle (42) et indirectement la seconde unité fonctionnelle (44) peuvent être commandées électriquement ou électroniquement par actionnement d'un premier élément d'actionnement (S1) qui est prévu sur la première unité d'appareil (12) ; et
à l'état découplé des deux unités d'appareil (12, 14), la section de couplage (66) de la première unité fonctionnelle (42) est amenée automatiquement dans la position de couplage (B) au moyen d'une énergie extérieure lorsque les deux unités d'appareil (12, 14) se trouvent dans la position relative déterminée l'une à l'autre ou n'ont pas atteint la distance minimale prédéterminée entre elles.

10. Appareil médical (10) selon l'une des revendications 5 à 8, **caractérisé en ce que**
à l'état couplé des deux unités d'appareil (12, 14), la première unité fonctionnelle (42) et indirectement la seconde unité fonctionnelle (44) peuvent être commandées électriquement ou électroniquement par actionnement d'un second élément d'actionnement (S2) qui est prévu sur la seconde unité fonctionnelle (44) ; et
le second élément d'actionnement (S2) est réalisé de telle manière, en particulier est réalisé comme contact à ouverture électrique de sorte que lors du couplage électrique des deux unités d'appareil (12, 14), l'apport d'énergie pour la commande de la première unité fonctionnelle (12) soit interrompue.

11. Appareil médical (10) selon l'une quelconque des revendications précédentes, **caractérisé en ce que** la distance minimale est inférieure ou égale à 40 mm, en particulier inférieure ou égale à 20 mm, de manière particulièrement préférée inférieure ou égale à 15 mm.

12. Procédé de couplage mécanique de deux unités fonctionnelles (42, 44) d'un appareil médical (10) à deux parties ou plus, dans lequel
l'appareil médical (10) présente deux unités d'appareil (12, 14) pouvant être couplées mécaniquement l'une à l'autre,
les deux unités d'appareil (12, 14) présentent deux unités fonctionnelles (42, 44) pouvant être couplées mécaniquement l'une à l'autre afin de pouvoir actionner, à l'état couplé mécaniquement avec la première unité fonctionnelle (42), la seconde unité fonctionnelle (44) ; et
la première unité fonctionnelle (42) est commandable électriquement ou électroniquement et présente une section de couplage mécanique (66) qui peut être amenée dans une position de couplage (B) pour le couplage avec une section de couplage (68) mécanique correspondante de la seconde unité fonctionnelle (44),
**caractérisé par** les étapes :
la détection d'une position relative ou d'une distance des deux unités d'appareil (12, 14) l'une par rapport à l'autre ; et
la configuration de la commande électrique ou électronique de la première unité fonctionnelle (42) de telle manière que la section de couplage mécanique (66) de la première unité fonctionnelle (42) ne soit amenée dans la position de couplage que lorsque les deux unités d'appareil (12, 14) se trouvent dans une position relative déterminée l'une par rapport à l'autre ou n'atteignent pas une distance minimale prédéterminée l'une de l'autre.
